Europäisches Patentamt

European Patent Office

Office européen des brevets

(19)

(11) Veröffentlichungsnummer: **0 043 447**
A2

(12) EUROPÄISCHE PATENTANMELDUNG

(21) Anmeldenummer: **81104285.2**

(22) Anmeldetag: **04.06.81**

(51) Int. Cl.³: **A 61 B 17/36**, A 61 M 19/00,
A 61 C 19/08

(30) Priorität: **13.06.80 DE 3022216**

(43) Veröffentlichungstag der Anmeldung: **13.01.82**
**Patentblatt 82/2**

(84) Benannte Vertragsstaaten: **AT BE CH FR GB IT LI LU NL**

(71) Anmelder: **Kreibel, Karl-Heinz,, Ostfeldstrasse 25,
D-3000 Hannover (DE)**
Anmelder: **Hennecke, Heinz Ing., An Der Autobahn 35,
D-3012 Langenhagen (DE)**

(72) Erfinder: **Kreibel, Karl-Heinz,, Ostfeldstrasse 25,
D-3000 Hannover (DE)**
Erfinder: **Hennecke, Heinz Ing., An Der Autobahn 35,
D-3012 Langenhagen (DE)**

(74) Vertreter: **Eikenberg & Brümmerstedt Patentanwälte,
Schackstrasse 1, D-3000 Hannover (DE)**

(54) Tieftemperatur-Übertragungseinrichtung für medizinische Zwecke.

(57) Es wird eine Tieftemperatur-Übertragungseinrichtung zur schmerzreduzierenden Behandlung von erkrankten Nerven, Gefäßen, Geweben, von Karies und Wunden durch punktuellen Kälteschock beschrieben. Die Tieftemperatur-Übertragungseinrichtung besteht aus einem Kältespeicher aus hochleitfähigem Material, der nach Abkühlung in einen isolierenden Behälter eingesetzt wird und dann mit einer aus diesem herausragenden Spitze mit der zu behandelnden Zone in Eingriff gebracht wird, um diese durch Abkühlung augenblicklich zu betäuben und damit die Zeit einzusparen, die bisher durch Benutzung von Betäubungsspritzen oder dergl. bis zum Eintritt der Wirkung abgewartet werden mußte.

81104285.2

Karl-Heinz Kreibel, Heinz Hennecke                    506/1 Eu


### Tieftemperatur-Übertragungseinrichtung
### für medizinische Zwecke


Die Erfindung betrifft eine Tieftemperatur-Übertragungseinrichtung zur schmerzreduzierenden Behandlung von
erkrankten Nerven, Gefäßen, Geweben, Karies und Wunden
durch punktuellen Kälteschock.

Hierbei wird durch das Aufsetzen einer entsprechend geformten Kälteleiterspitze (2) im Bereich des entzündeten Gewebes, der Nervenenden oder Gefäße eine spontane Vereisung bewirkt.

Bei der Kariesbehandlung an vitalen Zähnen kann
durch die punktuelle Kälteübertragung – mittels entsprechend
geformter Kälteleiterspitze (2) in Größe der befallenen
Zone – Schmerzunempfindlichkeit erreicht werden, so daß
auf Betäubungsspritzen verzichtet werden kann und somit
Nebenwirkungen bei medikamentempfindlichen Patienten vermieden werden.

Der Erfindung liegt die Aufgabe zugrunde, solche
Nebenwirkungen zu vermeiden und die bei der Benutzung von
Betäubungsspritzen erforderliche Zeit bis zur Wirkung einzusparen. Ferner ähnliche Anwendungen, z. B. im Bereich der Neurochirurgie und bei der Notwendigkeit partieller Nerven-Gewebe- oder Gefäßbehandlungen, zu ermöglichen.

Gelöst wird diese Aufgabe durch die Anwendung der Tieftemperatur-Übertragungseinrichtung, wobei durch Dosierung und Einwirkungsdauer, sowie die Wahl der Behandlungstemperatur, von der Nervenbetäubung über Gefäßkontraktion, Blutstillung, bis zum Veröden (Kristallisieren) von Sekreten und Gewebeteilen operiert werden kann.

Bei der Zahnbehandlung werden mit Objekttemperaturen von -60°C bis -90°C an der Kälteleiterspitze (2) brauchbare Resultate erzielt.

Die Behandlungsweise kann, je nach zu erzielendem Ergebnis, vom kurzen Antupfen eines Nerves bis zur völligen Zerstörung von Gefäßen, Gewebe oder Sekret durch längere Einwirkungsdauer erfolgen.

Gegenüber bekannten Operationsmethoden mittels Glühdraht (Kauter) ergibt sich der Vorteil, daß die Operationsrandzonen durch starke Unterkühlung bis Vereisung und Kontraktion betäubt oder ruhiggestellt werden können, dann der eigentliche Herd beseitigt werden kann und schließlich nach dem Wiederauftauen keine verbrannten Ränder vorhanden sind. Ein weiterer Vorteil liegt darin, daß das Operationsfeld durch die zum Stillstand gebrachte Blutung übersichtlicher wird.

0043447

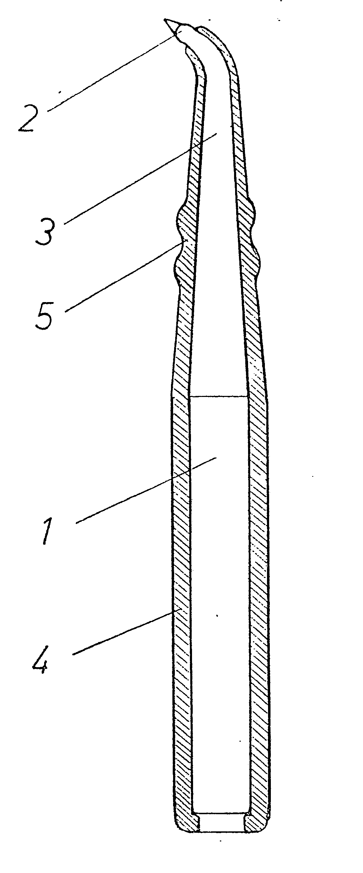

Patentansprüche :
------------------------------------

1.      Tieftemperatur-Übertragungseinrichtung zur schmerzreduzierenden Behandlung von Nerven, Zellgewebe, Gefäßen, Karies etc. durch Gefäßkontraktion bzw. Ruhigstellung bei kurzzeitiger Berührung, sowie zum partiellen Kristallisieren und Veröden erkrankter oder befallener Gewebeteile, Absonderungen und Sekretionen, durch dosierte oder totale Vereisung bei längerer Einwirkungsdauer und vorzugsweise Temperaturen unter -60°C, gekennzeichnet durch die folgenden Merkmale:

a) Kältespeicher (1) aus hochleitfähigem Material, z. B. Silber, Kupfer oder geeigneten Legierungen.

b) Operationsspitze (2) aus hochleitfähigem Material z. B. Edelmetall, mit vorzugsweise halbkugelig und anschließend spitz geformter, dem Behandlungsobjekt in der Größe entsprechender Form.

c) Kälteleiter (3) mit vorzugsweise konisch geformter, dem Behandlungsobjekt entsprechend gebogener Form, querschnittsmäßig für optimalen und spontanen Kälteübergang ausgelegt.

d) Isolierhandgriff (4), allseitig bis auf die Operationsspitze (2) kälteisoliert, gekennzeichnet durch Fingerstützen (5) und eine Handgrifform, die eine ausgewogene und sichere Handhabung bei der Anwendung ermöglicht.

2.      Einrichtung nach Anspruch 1, <u>dadurch gekennzeich-net,</u> daß der als Massivkörper (Kondensator) aus hochleitfähigem Material, z. B. Silber, ausgebildete Kältespeicher (1) zur Kälteübertragung zeitweise in ein Isoliergefäß (Dowar) eingelegt wird, welches mit einem Kältemittel von tiefer Temperatur z. B. Trockeneis oder Flüssiggas gefüllt ist.

3.      Einrichtung nach Anspruch 1, <u>dadurch gekennzeich-net,</u> daß der Kältespeicher (1) als Hohlkörper ausgebildet ist, der mit einem Kältemittel, z. B. einem bei tiefer Temperatur verflüssigten Gas, gefüllt ist.

4.      Einrichtung nach Anspruch 1, <u>dadurch gekennzeich-net,</u> daß  der Kältespeicher (1) als ein von Sole oder Kältemittel durchspülter Hohlkörper ausgebildet ist, bei welchem die Kälteübertragung über isolierte Schlauchleitungen und ein Tiefkühlaggregat erfolgt.

5.      Einrichtung nach Anspruch 1, <u>gekennzeichnet durch</u> die Ausführung der Operationsspitze (2) als Peltier-Element.

6.      Einrichtung nach Anspruch 1, <u>gekennzeichnet durch</u> Ausführung des  Isolierhandgriffes (4) als Kühler für das Peltier-Element.

0043447